# EUROPEAN PATENT APPLICATION

(11) **EP 2 206 464 A1**
(43) Date of publication of application: **14.07.2010**
(21) Application number: 10150501.4
(22) Date of filing: 12.01.2010
(51) Int. Cl.: A61B 5/16

(54) **Computerized test apparatus and methods for quantifying psychological aspects of human responses to stimuli**

(30) Priority: 12.01.2009 US 143948 P
(71) Applicant: Neuro - Technology Solutions Ltd., 76854 Kfar Mordechai (IL)
(72) Inventor: Yachin, Nir, 79241, Kfar Aviv (IL)
(74) Representative: Modiano, Micaela Nadia

(57) **Abstract**

A computerized method and apparatus for measuring effects of distraction on a human subject, including presenting at least one stimulus, at least one visual distraction and at least one audio distraction to a subject; measuring at least one aspect of at least one of the subject's commissions and omissions responsive to the stimulus; computing an indication of quality of at least one of the subject's commissions and omissions when visual distraction is present; and computing an indication of quality of at least one of the subject's commissions and omissions when audio distraction is present.

## Description

### REFERENCE TO CO-PENDING APPLICATIONS

Priority is claimed from U.S. Provisional Patent Application No. 61/143,948 entitled "Diagnostic test for ADHD" and filed January 12, 2009.

### FIELD OF THE INVENTION

The present invention relates generally to computerized testing apparatus and more particularly to computerized testing of cognitive functions,

### BACKGROUND OF THE INVENTION

Computerized testing systems for cognitive syndromes such as ADHD are known,

A well known class of computerized tools for diagnosing cognitive syndromes such as ADHD is the CPF which is a class of tests in which a child (e.g.) is asked to respond, typically by operating a computer input device e.g. by pressing and/or or not pressing certain keys on a computer keyboard, depending on the stimulus which appears on the computer's screen or other output device. Errors of omission are occurrences in which the child fails to respond (e.g. press a key) in response to a designated target stimulus's appearance. Errors of commission are occurrences in which the child responds, e.g. presses the key when the target stimulus is absent. Reaction time and its variability may also be computed.

The disclosures of all publications and patent documents mentioned in the specification, and of the publications and patent documents cited therein directly or indirectly, are hereby incorporated by reference.

### SUMMARY OF THE INVENTION

Certain embodiments of the present invention seek to provide computerized test apparatus for quantifying cognitive or other psychological aspects of a child or other subject's responses to stimuli.

The computerized test apparatus typically generates valid operationalizations of at least one of attentiveness, hyperactivity and, separately, impulsiveness, typically for more than one type of at least one of these.

Typically, a hyperactivity indication is generated if an end user generates more than the requested quantity, e.g. more than one, response to an individual stimulus, typically either while the stimulus is present or during a "void" period thereafter in which no stimulus is present.

Typically a first-type impulsiveness indication is generated if an end user generates too-quick and inappropriate responses e.g. if the end user's inappropriate or "bad" responses (typically - the end user's first response after appearance of a non-target stimulus on the display screen or other output device) are faster than his "good" or appropriate responses and/or if the end user's inappropriate responses are faster than the normal range of reaction time required to generate an appropriate response without guessing and/or if the end user responds during a time period in which s/he was instructed not to respond e.g. during a time period in which a non-target stimulus is being presented.

A particular feature of certain embodiments of the present invention is to generate hyperactivity indications and, by a different set of criteria, impulsiveness indications.

The apparatus is typically designed to allow a typical user e.g. child user to complete a test session without prematurely terminating his own performance.

The apparatus typically provides basic situations in which a target stimulus is presented to which the end user is instructed to respond by providing a computer input e.g. a space bar press, a non-target stimulus to which the end user has not been instructed to respond is presented, typically in the same screen location as the target stimulus, no stimulus is presented, a visual distraction such as a moving and/or pictorial element e.g. a video sequence is presented, an audio distraction is presented, and complex situations in which the basic situations are combined.

The term "distraction" as used herein is intended to include stimuli which are typically superimposed on or otherwise presented in association with a target and/or non-target stimuli and hence may affect, negatively or even positively, the end-user's ability to relate to, hence respond to, the target- and non-target stimuli toward which testing is focused.

Typically, the target and non-target stimuli are provided in a pre-defined screen location such as the approximate center of the screen. Typically, sequential appearances of target and non-target stimuli are separated by a "void" time period in which no stimulus appears, thereby to enable "void analysis" of the end user's behavior during the "void" time period and to thereby to generate outputs characterizing user's responsivity patterns. "Void analysis" typically includes analysis of good void presses and/or Bad Hyper Void presses as described herein.

Typically, a user action is considered to be a response to a stimulus which preceded it if the user action occurs while the stimulus is present on the computer screen or during the "void" time period which follows the period of presence of the stimulus on the screen.

Typically, stimuli are presented for longer time periods such as 3 seconds and for shorter time periods such as 0.5 seconds and users' responses to the longer-presented stimuli is compared to the same users' responses to the same stimuli when presented for a shorter length of time, thereby to obtain outputs characterizing user responsivity patterns.

Typically, a user's behavior subsequent to void time periods is analyzed to obtain outputs characterizing the user's responsivity patterns. For example, some users may perform poorly, subsequent to a void time period perhaps indicating a difficulty in "recovering" from periods of relaxation.

Typically, stimuli including distractions are presented to a user on a computer screen and/or using computer audio apparatus, data gathering is performed by computer tracking of a user's computer inputs e.g. space bar presses, and computational steps are performed by a computer in data association with the computer screen and with a user input device employed by the user to generate the computer inputs.

According to certain embodiments of the present invention, stimuli including distractions are pre-tested to ensure that a high proportion of end users finishes the test rather than terminating prematurely. It is believed that very tedious stimuli and/or distractions are not necessary to quantify most psychological characteristics of interest and are not desirable since these are believed to cause end users to terminate prematurely. It is believed that a changing environment is helpful in maintaining end user interest at a level which prevents premature termination by the user. For example, in level 2 a user may see a gong moving, in level 4 the user may hear the gong but not see it, and in level 6 the user both sees and hears the gong. Many other examples are possible.

According to certain embodiments of the invention, at least one target and/or non-target stimulus and/or distraction employed in the system of the present invention includes an audio-visual element e.g. sequence which is presented in at least two of the following forms: audio only, visual only, both audio and visual tracks.

According to certain embodiments of the invention, at least one target and/or non-target stimulus includes a first audio and/or visual element e.g. sequence and at least one distraction includes a second audio and/or visual element e.g. sequence and the system of the present invention displays the first and second elements, superimposed, to the user.

According to certain embodiments of the present invention, visual target and non-target stimuli to which the user is instructed to react and not react respectively, are always presented at a single pre-defined location on the computer screen.

According to certain embodiments of the present invention, at least one visual distraction includes at least one moving part and/or translates in its entirety within the computer screen e.g. birds which flap their wings and/or fly across the screen.

According to certain embodiments of the present invention, at least one stimulus and/or distraction includes a complex figure other than an alphanumeric character, such as a cartoon figure.

A particular advantage of certain embodiments of the present invention is that false positives and false negatives, such as children mis-identified as impulsive or hyperactive, or conversely failure to diagnose children who actually are impulsive or hyperactive, are prevented e.g. by not using letters which may prevent some end users such as dyslexics from succeeding; and/or by not requiring a user to observe geometric shapes that appear in different zones and press to indicate that these have entered a pre-defined section, so as to prevent errors regarding end user having visual perception handicaps.

Typically, the architecture of the code is constructed such that some or all display and other timing, graphics, element and distraction selection and sequencing, and element/distraction type parameters are all configurable. Typically, the core of the architecture includes Target elements, Non Target elements, and voids separating presentation of these.

According to certain embodiments of the present invention, stimuli and/or distractions are selected for presentation randomly from a library of stimuli and/or distractions. This is particularly useful when the testing system shown and described herein is integrated with a cognitive training system, typically including a data interface with the cognitive training system's software for providing an end user with feedback regarding a psychological aspect of his responses to stimuli and evaluating his attempts to utilize that feedback to improve that response aspect in her- or himself.

According to certain embodiments of the present invention, outputs are provided diagnosing presence in an end user of two types of impulsivity. A "Classic Impulsivity" output may be provided if at least one press is generated by the end user responsive to a threshold number of non target stimuli. A second type of impulsivity output may be provided if the average good press reaction time is found to differ from the average reaction time of impulse presses including the set of first presses responsive to non-target stimuli). It is believed that average good press reaction time represents a full thinking process and average impulse reaction time may be 20% - 40% less than the average good press reaction time. It is believed that a certain proportion of the impulsive users constitute a separate type of impulsives which have the same reaction time average for impulse presses and for good presses.

According to certain embodiments of the present invention, a global timer is provided that is initialized at the beginning of the test, and the time, relative to the global timer, of each press or non- press is stored. This is particularly advantageous if the testing system shown and described herein is integrated with an electro-physical measurement device such as an E.E.G or fMRI device. The result is a system for deeper evaluation because a user's impulsive action or hyperactive action can be correlated temporally to occasions on which the user's EEG or FMRI showed signs of brain activity.

According to certain embodiments of the present invention, target and non-target stimuli, and/or distractions thereto, are presented on the left and/or right of the screen, so as to generate outputs indicative of end-user attention deficits which may correlate specifically to the user's right or left side. Similarly, target and non-target stimuli, and/or distractions thereto, may be presented on the top and/or bottom of the screen, so as to generate outputs indicative of end-user attention deficits which may correlate specifically to, say, the top and bottom halves of a user's field of view. Target and non-target stimuli, and/or distractions thereto, may also be presented on both ends of a Z-axis or depth axis perpendicular to the axis of the computer screen, by generating 3D stimuli, so as to generate outputs indicative of end-user attention deficits pertaining to perception of one or another end of the Z-axis.

The present invention typically includes at least the following embodiments:

Embodiment 1. A method for measuring effects of distraction on a human subject, the method comprising:
presenting at least one stimulus, at least one visual distraction and at least one audio distraction to a subject;
measuring at least one aspect of at least one of the subject's commissions and omissions responsive to the stimulus;
computing an indication of quality of at least one of the subject's commissions and omissions when the visual distraction is present; and
computing an indication of quality of at least one of the subject's commissions and omissions when the audio distraction is present.

Embodiment 2. A method according to embodiment 1 and also comprising:
computing an indication of quality of at least one of the subject's commissions and omissions when the audio and visual distractions are both present.

Embodiment 3. A method according to any of embodiments 1 - 2 and also comprising using at least one of the indications to quantify attentiveness.

Embodiment 4. A method according to any of embodiments 1 - 3 and also comprising using at least one of the indications to quantify hyperactivity.

Embodiment 5. A computerized test system comprising:
apparatus for measuring a plurality of characteristics of responses generated by subjects;
a library of candidate syndromes, each candidate syndrome comprising a set of value ranges for at least some of the plurality of characteristics; and
a counter operative to accumulate a number of subjects answering to each candidate syndrome in the library and to provide a syndrome-identified output when the number of subjects answering to an individual candidate syndrome exceeds a predetermined threshold.

Embodiment 6. A system for measuring effects of distraction on a human subject, the system comprising:
a presentation generator operative to present to a subject at least one stimulus accompanied by at least one distraction selected from a universe of distractions including at least one visual distraction and at least one audio distraction; and
a performance measurement unit operative to measure the subject's test performance as affected by at least one audio distraction, to measure the subject's test performance as affected by at least one visual distraction, and to compare the two performances thereby to characterize the subject's differential response to audio and visual distractions.

Embodiment 7. A system according to embodiment 6 wherein the performance measurement unit is also operative to measure the subject's test performance as affected by at least one audio distraction in combination with at least one video distraction.

Embodiment 8. A system according to any of embodiments 6 - 7 wherein the performance measurement unit is also operative to measure at least one aspect of at least one of the subject's commissions and omissions responsive to the stimulus.

Embodiment 9. A system according to any of embodiments 6 - 8 wherein the performance measurement unit is also operative to compute an indication of quality of at least one of the subject's commissions and omissions when the visual distraction is present.

Embodiment 10. A system according to any of embodiments 6 - 9 wherein the performance measurement unit is also operative to compute an indication of quality of at least one of the subject's commissions and omissions when the audio distraction is present.

Embodiment 11. A system according to any of embodiments 6 - 10 wherein the universe of distractions includes a plurality of visual distractions with a corresponding plurality of profiles of visual characteristics.

Embodiment 12. A system according to any of embodiments 6 - 11 wherein the universe of distractions includes a plurality of audio distractions with a corresponding plurality of profiles of audio characteristics.

Embodiment 13. A system according to embodiment 11 wherein the performance measurement unit is operative to compare the subject's performance as affected by a visual distraction with a first profile of visual characteristics, with the subject's performance as affected by a visual distraction with a second, different profile of visual characteristics.

Embodiment 14. A system according to embodiment 12 wherein the performance measurement unit is operative to compare the subject's performance as affected by a audio distraction with a first profile of audio characteristics, with the subject's performance as affected by a audio distraction with a second, different profile of audio characteristics.

Embodiment 15. A method for measuring effects of distraction on a human subject, the method comprising:
presenting at least one stimulus to a subject;
generating a reaction time parameter representing the subject's reaction time and measuring at least one aspect of at least one of the subject's commissions and omissions responsive to the stimulus, other than reaction time;
generating an output characterizing the subject as a function of the reaction time parameter and of the aspect other than reaction time.

Embodiment 16. A method according to embodiment 15 and also comprising measuring the subject's reaction time responsive to a set of stimuli which is not identical to a set of stimuli including only the stimulus, and wherein the reaction time parameter is computed as a function of the subject's reaction time responsive to the stimulus, relative to the subject's reaction time responsive to the set of stimuli.

Embodiment 17. A method according to embodiment 16 wherein the set of stimuli comprises an empty set (void).

Embodiment 18. A method for measuring a human subject's performance as a function of at least one stimulus, the method comprising:
measuring the subject's performance responsive to presence of at least one stimulus displayed on a computer screen thereby to generate a first performance output;
measuring the subject's performance responsive to absence of the at least one stimulus from the computer screen thereby to generate a second performance output; and
comparing the first and second first performance outputs thereby to generate a characterization of the human subject's performance as a function of the at least one stimulus.

Embodiment 19. A method according to embodiment 18 and also comprising:
presenting to a subject at least one stimulus accompanied by at least one distraction selected from a universe of distractions; and
measuring the subject's performance as affected by at least one distraction, and comparing the two performances.

Embodiment 20. A system for measuring effects of stimuli on a human subject, the system comprising:
a delayed response monitor operative to monitor a subject's tendency to respond to a stimulus after the stimulus has terminated.

Embodiment 21. A method according to any of embodiments 1 - 4 and also comprising using at least one of the indications to quantify impulsiveness.

Embodiment 22. A method according to embodiment 15 and wherein the aspect other than reaction time comprises at least one of:
an aspect of the order of the subject's commissions and omissions;
an aspect of the relative timing of the subject's commissions and omissions; and
timing of commissions and omissions relative to a void time period in which the stimulus is not presented to the subject.

Embodiment 23. A cognitive testing system in which stimuli and/or distractions are selected for presentation at least partly randomly from a library of stimuli and/or distractions, which system is integrated with a cognitive training system providing an end user with feedback regarding a psychological aspect of his responses to the stimuli including responses in the presence of at least one distraction.

Embodiment 24. A cognitive testing system in which stimuli and/or distractions are selected for presentation to a user and a global timer is provided that is initialized at the beginning of the test, and the time, relative to the global timer, of each user response, generated using a computerized input device, is stored.

Embodiment 25. A system according to embodiment 24 which is integrated with a brain activity monitoring device such as an EEG or fMRI device.

Also provided is a computer program product, comprising a computer usable medium or computer readable storage medium, typically tangible, having a computer readable program code embodied therein, said computer readable program code adapted to be executed to implement any or all of the methods shown and described herein. It is appreciated that any or all of the computational steps shown and described herein may be computer-implemented. The operations in accordance with the teachings herein may be performed by a computer specially constructed for the desired purposes or by a general purpose computer specially configured for the desired purpose by a computer program stored in a computer readable storage medium.

Any suitable processor, display and input means may be used to process, to display e.g. on a computer screen or other computer output device which may for example comprise a platform that can render flash technology, to store, and to accept information such as information used by or generated by any of the methods and apparatus shown and described herein; the above processor, display and input means including computer programs, in accordance with some or all of the embodiments of the present invention. Any or all functionalities of the invention shown and described herein may be performed by a conventional personal computer processor, workstation or other programmable device or computer or electronic computing device, either general-purpose or specifically constructed, used for processing; a computer display screen and/or printer and/or speaker for displaying; machine-readable memory such as optical disks, CDROMs, magnetic-optical discs or other discs; RAMs, ROMs, EPROMs, EEPROMs, magnetic or optical or other cards, for storing, and keyboard or mouse, or any other device, having a communication capability e.g. via a flash platform through an appropriate API (Application Program Interface), for accepting. The term "process" as used above is intended to include any type of computation or manipulation or transformation of data represented as physical, e.g. electronic, phenomena which may occur or reside e.g. within registers and /or memories of a computer.

The above devices may communicate via any conventional wired or wireless digital communication means, e.g. via a wired or cellular telephone network or a computer network such as the Internet.

The apparatus of the present invention may include, according to certain embodiments of the invention, machine readable memory containing or otherwise storing a program of instructions which, when executed by the machine, implements some or all of the apparatus, methods, features and functionalities of the invention shown and described herein. Alternatively or in addition, the apparatus of the present invention may include, according to certain embodiments of the invention, a program as above which may be written in any conventional programming language, and optionally a machine for executing the program such as but not limited to a general purpose computer which may optionally be configured or activated in accordance with the teachings of the present invention. Any of the teachings incorporated herein may wherever suitable operate on signals representative of physical objects or substances.

The embodiments referred to above, and other embodiments, are described in detail in the next section.

Any trademark occurring in the text or drawings is the property of its owner and occurs herein merely to explain or illustrate one example of how an embodiment of the invention may be implemented.

Unless specifically stated otherwise, as apparent from the following discussions, it is appreciated that throughout the specification discussions, utilizing terms such as, "processing", "computing", "estimating", "selecting", "ranking", "grading", "calculating", "determining", "generating", "reassessing", "classifying", "generating", "producing", "stereo-matching", "registering", "detecting", "associating", "superimposing", "obtaining" or the like, refer to the action and/or processes of a computer or computing system, or processor or similar electronic computing device, that manipulate and/or transform data represented as physical, such as electronic, quantities within the computing system's registers and/or memories, into other data similarly represented as physical quantities within the computing system's memories, registers or other such information storage, transmission or display devices. The term "computer" should be broadly construed to cover any kind of electronic device with data processing capabilities, including, by way of nonlimiting example, personal computers, servers, computing system, communication devices, processors (e.g. digital signal processor (DSP), microcontrollers, field programmable gate array (FPGA), application specific integrated circuit (ASIC), etc.) and other electronic computing devices.

The present invention may be described, merely for clarity, in terms of terminology specific to particular programming languages, operating systems, browsers, system versions, individual products, and the like. It will be appreciated that this terminology is intended to convey general principles of operation clearly and briefly, by way of example, and is not intended to limit the scope of the invention to any particular programming language, operating system, browser, system version, or individual product.

### BRIEF DESCRIPTION OF THE DRAWINGS

Certain embodiments of the present invention are illustrated in the following drawings:
Fig. 1A is a simplified functional block diagram of a computerized test system constructed and operative in accordance with certain embodiments of the present invention.
Fig. 1B is a simplified flowchart illustration of a method for measuring effects of distraction on a human subject which may be implemented using the system of Fig. 1A, all in accordance with a first embodiment of the present invention.
Fig. 1C is a simplified flowchart illustration of a method for measuring effects of distraction on a human subject which may be implemented using the system of Fig. 1A, all in accordance with a second embodiment of the present invention.
Fig. 1D is a simplified flowchart illustration of a method for measuring a human subject's performance as a function of at least one stimulus, using the system of Fig. 1 A, all in accordance with a third embodiment of the present invention.
Fig. 1E is a simplified flowchart illustration of a method for measuring effects of distraction on a human subject, using the system of Fig. 1A, all in accordance with a fourth embodiment of the present invention.
Fig. 2A - 2C which are simplified pictorial illustrations of screens occurring during a computerized test generated by an example runtime environment constructed and operative in accordance with certain embodiments of the present invention.
Figs. 3A - 3F are simplified pictorial illustrations of pictorial elements any or all of which may serve as non-target stimuli in accordance with certain embodiments of the present invention.
Fig. 4 is a table showing an example sequence of Sublevels included in an individual level of testing in accordance with certain embodiments of the present invention.
Figs. 5 - 10B are example excerpts of movie clips any or all of which may serve as visual distractions in accordance with certain embodiments of the present invention.
Fig. 11 is a table setting out example timing and positioning of distractions on the computer screen in accordance with certain embodiments of the present invention.
Fig. 12 is a state diagram defining possible states which may be defined over an end user's performance in accordance with certain embodiments of the present invention.
Fig. 13A is a CurSubLvlResults table which may be constructed in computer memory for each sub level in accordance with certain embodiments of the present invention.
Fig. 13B is a lvlResults table which may be constructed in computer memory in accordance with certain embodiments of the present invention.
Fig. 13C is a mivRawResults table which may be constructed in computer memory in accordance with certain embodiments of the present invention.
Figs. 14A - 14B, taken together, are an example of a table, also termed herein the mivSummaryByLvl table, useful for presenting information about psychological variables such as Attention, hyperactivity and impulsiveness, which may be constructed in computer memory in accordance with certain embodiments of the present invention.
Fig. 15 is a table, termed herein the mivTopSummary table, which is similar to the table of Figs. 14A - 14B except that the grades are made from the top point of view of the test.
Fig. 16 is a Types & Subtypes table which may be constructed in computer memory in accordance with certain embodiments of the present invention.
Figs. 17A - 17D are pictorial illustrations of screenshots respectively corresponding to a 4-tab summary of outputs generated by the system shown and described herein, in accordance with certain embodiments of the present invention.
Figs. 18A - 18C are pictorial illustrations of output screen displays exemplifying another set of outputs generated by the system shown and described herein, in accordance with certain embodiments of the present invention.
Fig. 19 is a simplified functional block diagram of a computerized test system integrated with an electro-physical measurement device in accordance with certain embodiments of the present invention.

### DETAILED DESCRIPTION OF CERTAIN EMBODIMENTS

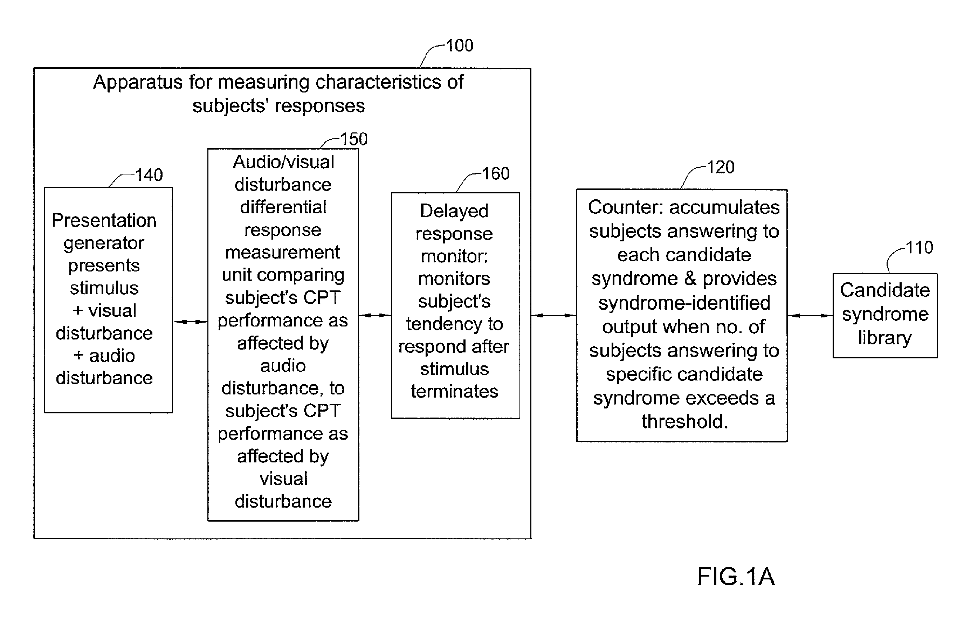

Fig. 1A is a simplified functional block diagram of a computerized test system comprising apparatus 110 for measuring a plurality of characteristics of responses generated by subjects; a library 110 of candidate syndromes, each candidate syndrome comprising a set of value ranges for at least some of said plurality of characteristics; and a counter 120 operative to accumulate a number of subjects answering to each candidate syndrome in the library and to provide a syndrome-identified output when the number of subjects answering to an individual candidate syndrome exceeds a predetermined threshold. Typically, the apparatus 110 includes a CPF test administrator operative for measuring effects of distraction on a human subject. The apparatus 110 may for example comprise a presentation generator 140 operative to present to a subject at least one stimulus accompanied by at least one distraction selected from a universe of distractions including at least one visual distraction and at least one audio distraction; and a performance measurement unit 150 operative to measure the subject's test performance as affected by at least one audio distraction, to measure the subject's test performance as affected by at least one visual distraction, and to compare the two performances thereby to characterize the subject's differential response to audio and visual distractions. The performance measurement unit 150 is typically also operative:
(a) to measure the subject's test performance as affected by at least one audio distraction in combination with at least one video distraction; and/or
(b) to measure at least one aspect of at least one of the subject's commissions and omissions responsive to said stimulus; and/or
(c) to compute an indication of quality of at least one of the subject's commissions and omissions when said visual distraction is present; and/or
(d) to compute an indication of quality of at least one of the subject's commissions and omissions when said audio distraction is present.

The universe of distractions may include a plurality of visual distractions with a corresponding plurality of profiles of visual characteristics and/or may include a plurality of audio distractions with a corresponding plurality of profiles of audio characteristics.

Typically, the performance measurement unit 150 is operative to compare the subject's performance as affected by a visual distraction with a first profile of visual characteristics, with the subject's performance as affected by a visual distraction with a second, different profile of visual characteristics and/or, similarly, to compare the subject's performance as affected by a audio distraction with a first profile of audio characteristics, with the subject's performance as affected by a audio distraction with a second, different profile of audio characteristics.

The apparatus 110 may include a delayed response monitor 160 operative to monitor a subject's tendency to respond after a stimulus has terminated.

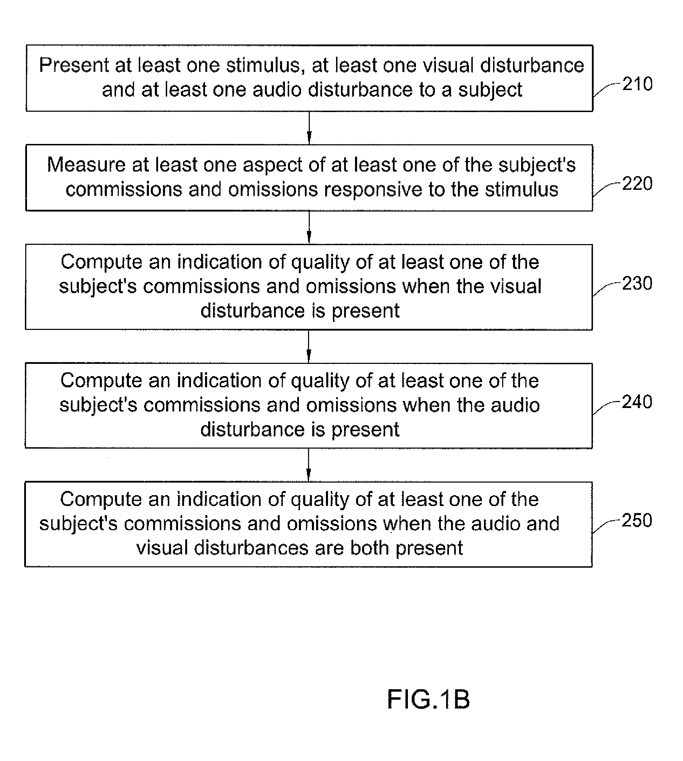

Fig. 1B is a simplified flowchart illustration of a method for measuring effects of distraction on a human subject which may be implemented using the system of Fig. 1A, all in accordance with a first embodiment of the present invention. The method of Fig. 1B may include some or all of the following steps, suitably ordered e.g. as shown:
presenting at least one stimulus, at least one visual distraction and at least one audio distraction to a subject;
measuring at least one aspect of at least one of the subject's commissions and omissions responsive to said stimulus;
computing an indication of quality of at least one of the subject's commissions and omissions when said visual distraction is present;
computing an indication of quality of at least one of the subject's commissions and omissions when said audio distraction is present; and
computing an indication of quality of at least one of the subject's commissions and omissions when said audio and visual distractions are both present.

The method of Fig. 1B may or may not generate a quantification of attentiveness and/or hyperactivity.

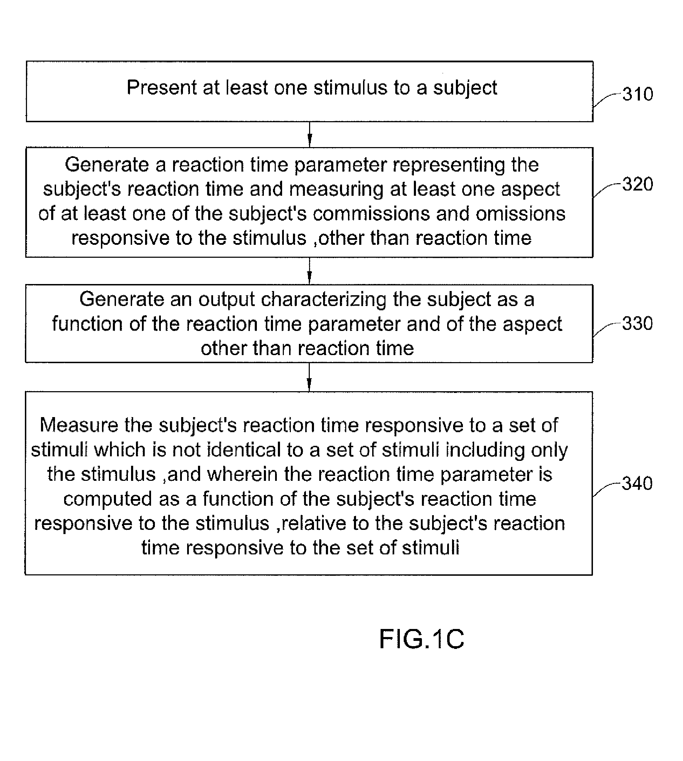

Fig. 1C is a simplified flowchart illustration of a method for measuring effects of distraction on a human subject which may be implemented using the system of Fig. 1A, all in accordance with a second embodiment of the present invention. The method of Fig. 1C may include some or all of the following steps, suitably ordered e.g. as shown:
presenting at least one stimulus to a subject;
generating a reaction time parameter representing the subject's reaction time and measuring at least one aspect of at least one of the subject's commissions and omissions responsive to the stimulus, other than reaction time;
generating an output characterizing the subject as a function of the reaction time parameter and of the aspect other than reaction time; and
measuring the subject's reaction time responsive to a set of stimuli which is not identical to a set of stimuli including only the stimulus, and wherein the reaction time parameter is computed as a function of the subject's reaction time responsive to the stimulus, relative to the subject's reaction time responsive to the set of stimuli.

It is appreciated that the set of stimuli may optionally comprise an empty set (void).

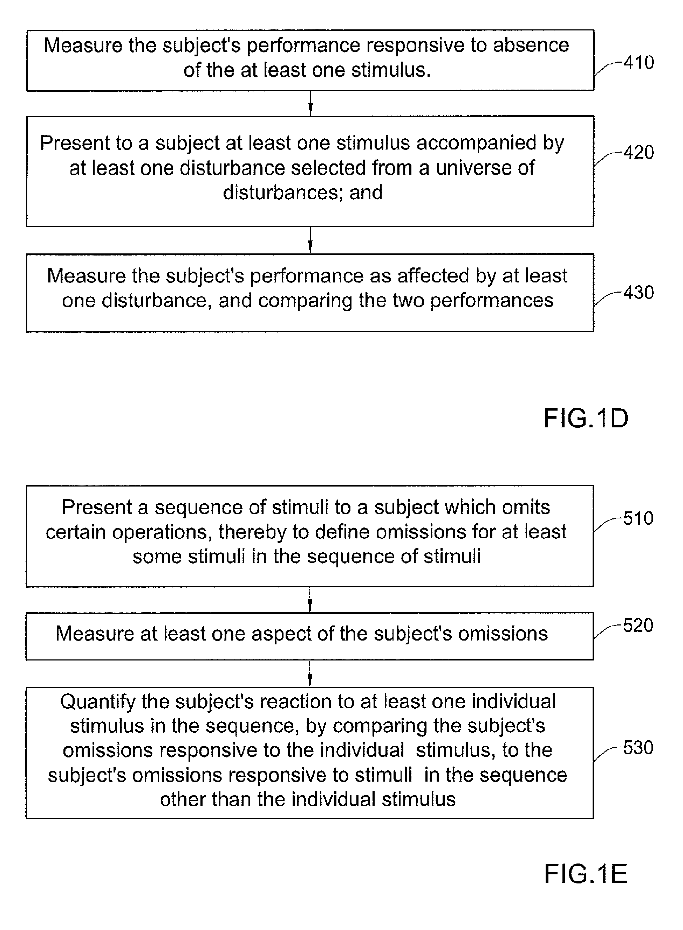

Fig. 1D is a simplified flowchart illustration of a method for measuring a human subject's performance as a function of at least one stimulus, using the system of Fig. 1A, all in accordance with a third embodiment of the present invention. The method of Fig. 1D may include some or all of the following steps, suitably ordered e.g. as shown:
measuring the subject's performance responsive to absence of the at least one stimulus;
presenting to a subject at least one stimulus accompanied by at least one distraction selected from a universe of distractions; and
measuring the subject's performance as affected by at least one distraction, and comparing the two performances.

Fig. 1E is a simplified flowchart illustration of a method for measuring effects of distraction on a human subject, using the system of Fig. 1A, all in accordance with a fourth embodiment of the present invention. The method of Fig. 1E may include some or all of the following steps, suitably ordered e.g. as shown:
presenting a sequence of stimuli to a subject which omits certain operations, thereby to define omissions for at least some stimuli in the sequence of stimuli;
measuring at least one aspect of the subject's omissions; and
quantifying the subject's reaction to at least one individual stimulus in the sequence, by comparing the subject's omissions responsive to said individual stimulus, to the subject's omissions responsive to stimuli in the sequence other than the individual stimulus.

An example of a Runtime environment and corresponding Analysis functionality useful in implementing various of the embodiments of Figs. 1A - 1E is now described.

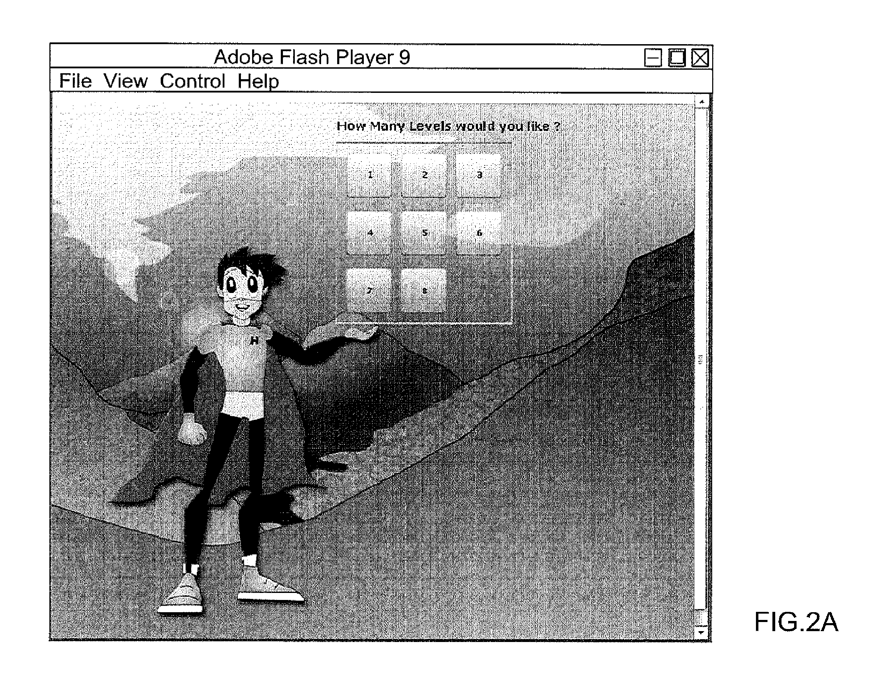

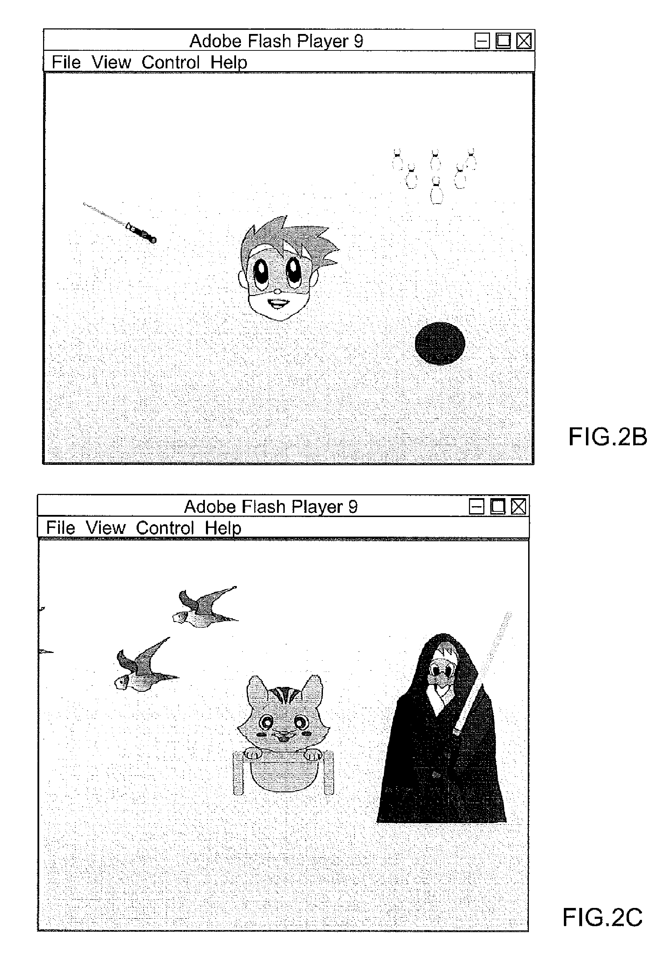

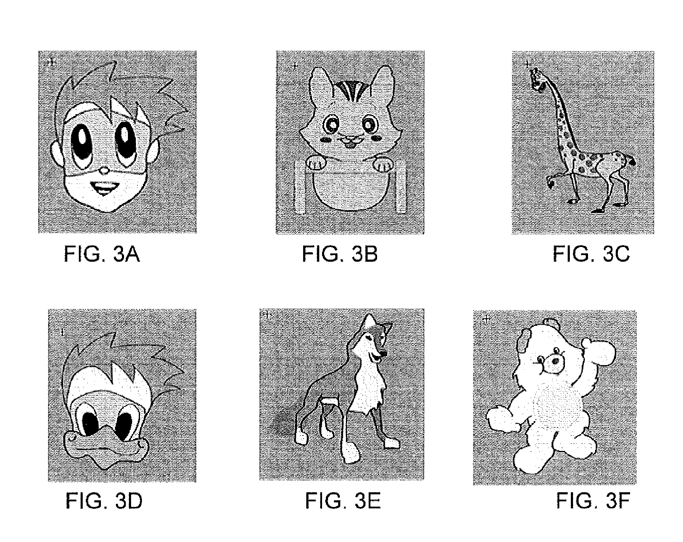

In the Runtime environment shown and described herein, the User is typically active, and the application gather information from the user's activities. The environment typically initially generates a welcome screen e.g. as shown in Fig. 2A, that enables the user to fill in forms and initial details and presents a fanciful figure termed herein "hyper" who may himself be a superhero with attention and organization problems. The environment then generates a computerized test. During the test the examinee is instructed to press one time on the spacebar only when the face of hyper is on the screen in the middle e.g. as shown in Fig. 2B. The instruction may for example be "Press the spacebar one time only when hyper's face is shown in the middle of the screen". In the illustrated embodiment, only the face of "hyper" is shown in the CPF to minimize the screen-space that the child is to pay attention to and maximize space for appearance of distractions. There are typically several e.g. eight levels in the test, which are presented one after the other. Each level typically includes a Measurable element system and a Distractions system. Every level has the same group of "measurable elements", typically shown on in the center of the screen in the same order. The group comprises the face of hyper and a variety of different animals, in the illustrated embodiment, e.g. as shown in Figs. 2B and 2C which are snapshots of screens occurring during a computerized test generated by the runtime environment shown and described herein. The group of measurable elements includes 6 measurable Elements in the illustrated embodiment, each element comprising a suitable pictorial element, e.g. as shown in Figs. 3A - 3F. El 1, the element shown in Fig. 3A, is the face of "hyper", and is also termed herein the "target" since this is the element which the subject has been instructed to react to. Elements 2 - 6 in the illustrated embodiment are Cat, Giraffe, Duck, Wolf and Bear respectively as shown. These elements are considered measurable because all user's actions and inactions or omissions are typically measured and analyzed relative to the element shown on screen during keyboard press time.

The number of times the spacebar was pressed during each show time of the above "measurable elements" is measured, where "show time" refers to the duration of time for which each measurable element is displayed to the child end user on the screen. Additionally the time which elapses from element appearance until the initial spacebar press, or until each spacebar press, is also typically accumulated and stored.

An example computerized test is about 15 minutes and includes 8 levels, each one having a 114 sec duration. Each such level may feature a different set of distractions that vary in their audio and/or video characteristics, e.g. as follows:
Level 1 - no distraction
Level 2 - 1 visual distraction
Level 3 - 2 visual distractions
Level 4 - 1 audio distraction
Level 5 - 2 audio distractions
Level 6 - one audio-visual distraction
Level 7 - two audio-visual distractions
Level 8 - no distraction.

Typically, visual distractions may comprise the visual component of one or more of the audio-visual distractions and audio distractions may comprise the sound track of one or more of the audio-visual distractions. For example, when a Visual distraction only is provided, one or more movie clips are shown and the sound is off. When an Audio distraction only is provided, one or more sound clips are on, but all movie clips are off.
When both Visual and Audio distractions are provided, sound is on and clips are shown.

A particular feature of certain embodiments of the present invention is isolating types of distraction e.g. audio and/or video, in order to identify which distraction or combination thereof, impairs or alternatively improves the child end-user's ability to follow the instructions given to her or him. Typically, grades and presses are compared between the different levels and performance patterns are sought after, all as described herein. The distraction Sequence may be the same for all the levels.

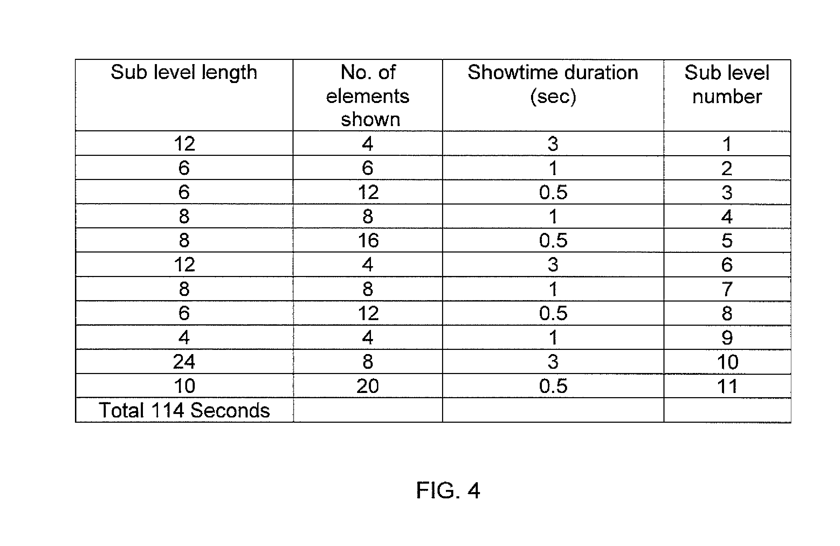

Fig. 4 is a table showing an example sequence of Sublevels included in an individual level. The sequence of the elements is, in the illustrated embodiment, the same for each level and includes 11 sublevels as shown in the table of Fig. 4. In each sublevel the show time duration of each element is the same (3 sec for sublevels 1, 6 and 10; 1 sec for sublevels 2, 4, 7 and 9; and 0.5 sec for sublevels 3, 5, 8 and 11, in the illustrated embodiment).

Suitable sets of 4, 6, 12, 8, 16, ... 20 (second column in Fig. 4) elements which may be shown in sub-levels 1, 2, 3, 4, 5, ... 11 are as follows, merely by way of example of course:

| Sub level 1 | |
|---|---|
| 1. | El 1 |
| 2. | - (the symbol "-" indicates that no measurable element appears) |
| 3. | El 1 |
| 4. | - |

| Sub level 2 | |
|---|---|
| 1. | El 1 |
| 2. | - |
| 3. | El 3 |
| 4. | - |
| 5. | El 1 |
| 6. | - |

| Sub level 3 | |
|---|---|
| 1. | El 2 |
| 2. | - |
| 3. | El 1 |
| 4. | - |
| 5. | El 4 |
| 6. | - |
| 7. | El 2 |
| 8. | - |
| 9. | El 1 |
| 10. | - |
| 11. | El 1 |
| 12. | - |

| Sub level 4 | |
|---|---|
| 1. | El 4 |
| 2. | - |
| 3. | El 1 |
| 4. | - |
| 5. | El 1 |
| 6. | - |
| 7. | El 1 |
| 8. | - |

| Sub level 5 | |
|---|---|
| 1. | El 3 |
| 2. | - |
| 3. | El 1 |
| 4. | - |
| 5. | El 6 |
| 6. | - |
| 7. | El 5 |
| 8. | - |
| 9. | El 1 |
| 10. | - |
| 11. | El 1 |
| 12. | - |
| 13. | El 3 |
| 14. | - |
| 15. | El 1 |
| 16. | - |

| Sub level 6 | |
|---|---|
| 1. | El 1 |
| 2. | - |
| 3. | El 1 |
| 4. | - |

| Sub level 7 | |
|---|---|
| 1. | El 5 |
| 2. | - |
| 3. | El 1 |
| 4. | - |
| 5. | El 3 |
| 6. | - |
| 7. | El 1 |
| 8. | - |

| Sub level 8 | |
|---|---|
| 1. | El 1 |
| 2. | - |
| 3. | El 1 |
| 4. | - |
| 5. | El 6 |
| 6. | - |
| 7. | El 3 |
| 8. | - |
| 9. | El 1 |
| 10. | - |
| 11. | El 1 |
| 12. | - |

| Sub level 9 | |
|---|---|
| 1. | El 1 |
| 2. | - |
| 3. | El 2 |
| 4. | El 1 |

| Sub level 10 | |
|---|---|
| 1. | - |
| 2. | El 5 |
| 3. | - |
| 4. | El 1 |
| 5. | - |
| 6. | 1 |
| 7. | - |
| 8. | El 2 |

| Sub level 11 | |
|---|---|
| 1. | - |
| 2. | El 1 |
| 3. | - |
| 4. | El 1 |
| 5. | - |
| 6. | El 1 |
| 7. | - |
| 8. | El 5 |
| 9. | - |
| 10. | El 2 |
| 11. | - |
| 12. | El 4 |
| 13. | - |
| 14. | El 1 |
| 15. | - |
| 16. | El 3 |
| 17. | El 1 |
| 18. | - |
| 19. | El 1 |
| 20. | - |

Typically the visual distractions' movie clips are superimposed onto the element sequence.
On levels 3, 5 and 7, in parallel to the first distraction, another, different, distraction is additionally shown on screen in parallel to the first.

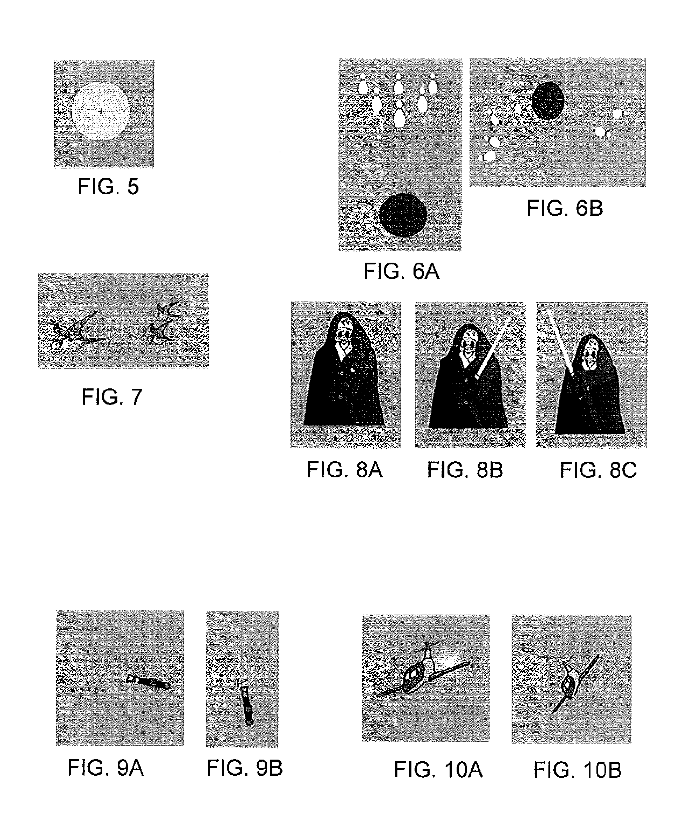

Examples of movie clips which may serve as visual distractions are shown in Figs. 5 - 10B.

Fig. 5 is a screenshot from a movie clip termed herein Ec1 or Gong in which a yellow circle shrinks and expands for a total of 6.8 Sec.

Figs. 6A - 6B are initial and final screenshots from a 3.5 Sec movie clip termed herein Ec2 or Bowling which starts with a bowling ball at the bottom of screen and standing pins; the ball goes up, hits the pins and spreads them to the sides.

Fig. 7 is a screenshot from a 9.25 Sec movie clip termed herein Ec3 or Birds in which three birds move from left to right and slightly wave their wings.

Figs. 8A - 8C are initial, interim and final screenshots from a 14.8 Sec movie clip termed herein Ec4 or Jedi which starts with a character called Jedi holding the handle of a closed saber; after a few seconds the saber is turned on and the character swings the saber from left to right and back to left then closes the saber.

Figs. 9A - 9B are initial and final screenshots from a 6.8 Sec movie clip termed herein Ec5 or Saber including a saber moving up, down, left and right.

Figs. 10A - 10B are initial and final screenshots from a 8.4 Sec movie clip termed herein Ec6 or Plane depicting an airplane that starts flying from the top right corner to the left; during its flight there is a small explosion at the plane's back end which does not disturb the plane flying.

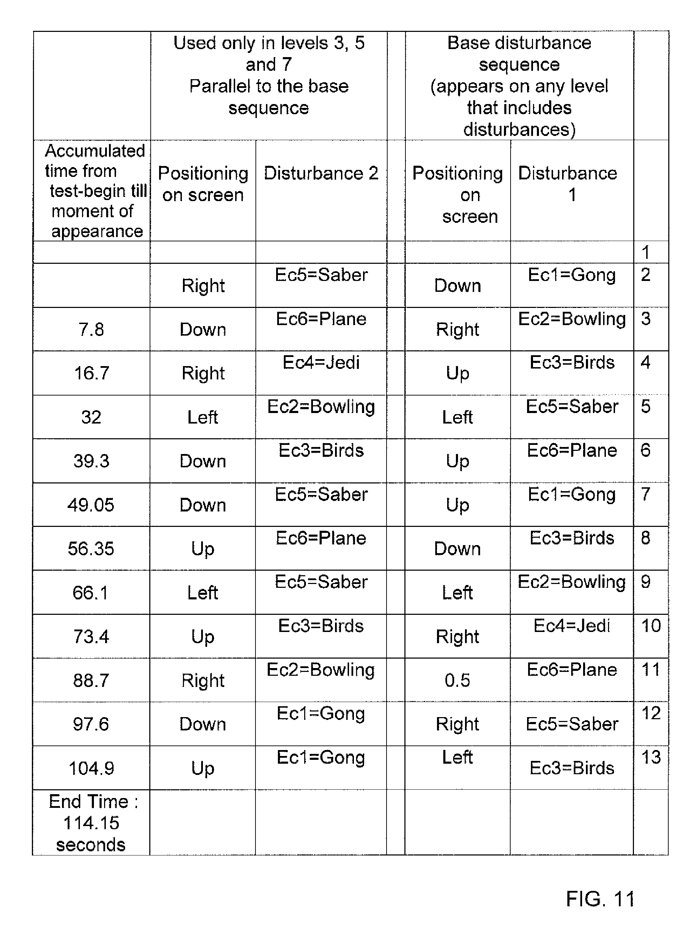

Timing and positioning of distractions on the computer screen may for example be as set out in the table of Fig. 11, Between each two distractions, i.e. between adjacent rows in the table of Fig. 11, 0.5 seconds may elapse.

Typical computations performed by differential response measurement unit 150 of Fig. 1 are now described. In the forgoing description, Hyperface is regarded as the Target stimulus and all other measurable elements are termed "Non Target". The "starting point" is the appearance time of the first target. There are no consecutive targets in the illustrated embodiment; each target is followed by a void element. The term "No press" refers to a situation in which the child end-user generates no presses until end of show time for a current measurable element. For the duration of appearance of a particular target, any of several performance outcomes are possible, such as the following 8 outcomes or states:
1. Good Press (GP)
2. Good Void Press (GVP)
3. Missed Press (MP)
4. Bad Hyper Press (BHP)
5. Bad Hyper Void (BHV)
6. Bad Press (BP)
7. Bad Animal Press (BAP)

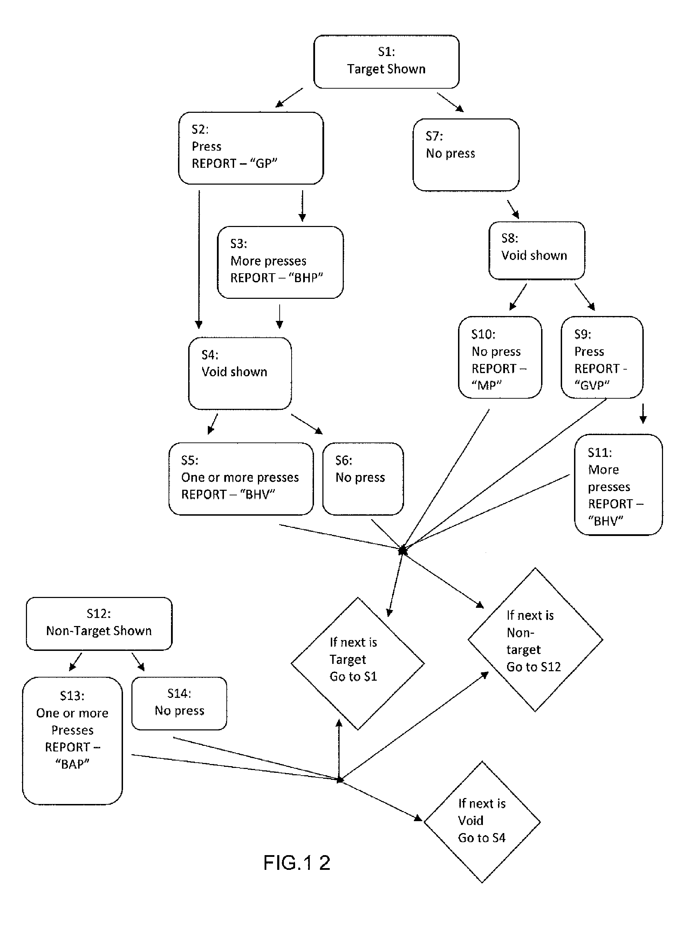

The above states are defined by the state chart of Fig. 12 which includes the following states and transitions therebetween:
S1: Target Shown on screen.
   If press go to →S2
   If no press go to → S8
S2: Report - "GP"
   If more presses go to → S3
   If void shown on screen go to → S4
S3: Report - "BHP" go to → S4
S4: Void on screen
   If one or more presses go to S5
   If no presses go to S6
S5: Report -"BHV"
   If next element is Target go to S 1
   If next element is Non-target go to S12
S6: If next element is Target go to S1
   If next element is Non-target go to S 12
S7: wait for void then go to S8
S8: Void on screen
   If press go to S9
   If no press go to S10
S9: Report -"GVP"
   If more presses go to S 11
   If next element is Target go to S 1
   If next element is Non-target go to S12
S10: Report -"MP"
   If next element is Target go to S 1
   If next element is Non-target go to S12
S 11: Report -"BHV"
   If next element is Target go to S 1
   If next element is Non-target go to S12
S12: Non Target on screen
   If one or more presses go to S13
   If no press go to S 14
S 13: REPORT - "BAP"
   If next element is Void go to S4
   If next element is Target go to S 1
   If next element is Non-target go to S12
S 14: If next element is Void go to S4
   If next element is Target go to S1
   If next element is Non-target go to S12

Bad Press (BP) is a state in which a user presses a key other than the spacebar (regardless of what is on the screen).

An analysis functionality is now described; typically, when this functionality is in operation, the user may be passive, whereas the system analyzes the data collected above and sends results of its analysis to a suitable server processor. During runtime the application gathers info about action/inaction of the user, typically at each of the following junctures:
a. On keyboard press - Each press is analyzed to determine the press status by predefined rules of actions e.g. as defined in Fig. 12
b. Every sub-level starting point.
c. Every ending point of measurable element show time.

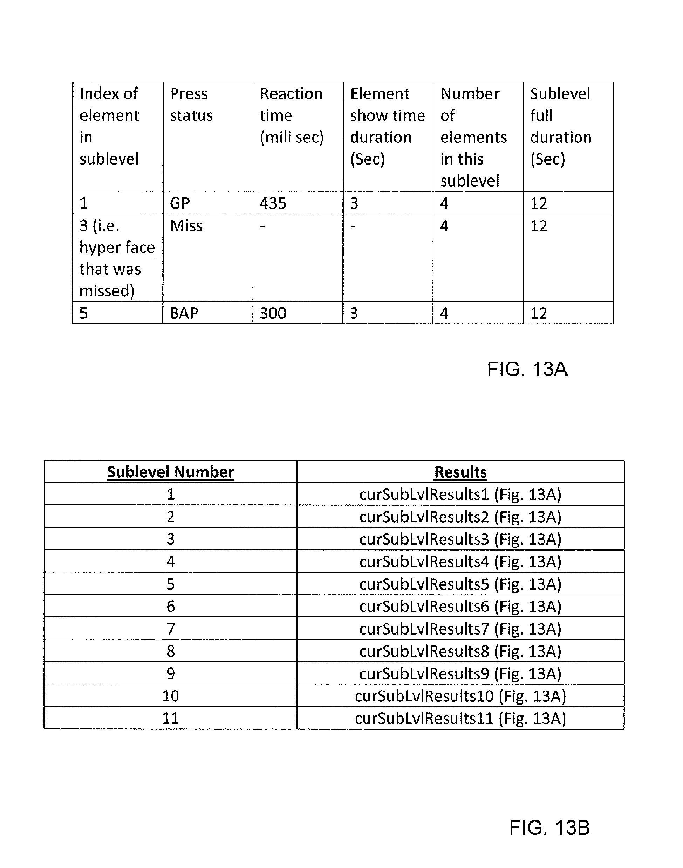

The following fields of information are stored in the table of Fig. 13A, also termed herein the CurSubLvlResults table, for each sub level i.e. 11 total per level in the illustrated embodiment:
A. Index of element in sublevel
B. Press status as defined above
C. RT (Reaction time), i.e. the time elapsed) in milliseconds from element appearance till spacebar pressing occurs
D. Element show time duration.
E. number of elements in current sublevel
F. Sublevel full duration, in Sec

The table of Fig. 13B, also termed herein the lvlResults or "actual data" table, stores all previous curSubLvlResults tables (11 tables) and at the End point of each Sub Level, the table of Fig. 13B is updated. The table of Fig. 13B is generated for every level such that 8 such tables are generated and stored in the illustrated embodiment.

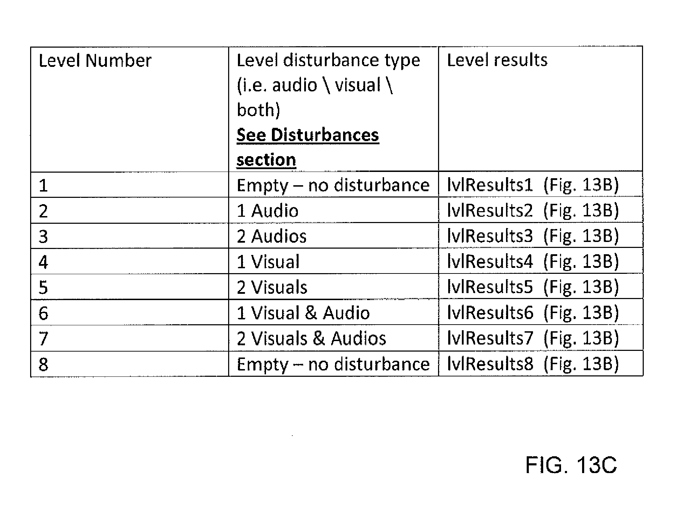

The table of Fig. 13C, also termed herein the mivRawResults table, is generated at the end of the test and is parsed by an analyzer as described in detail below. For every level in the test, a check is made of the reaction time of each press, including averaging, and suitable grades, texts and graphs may be generated as described.

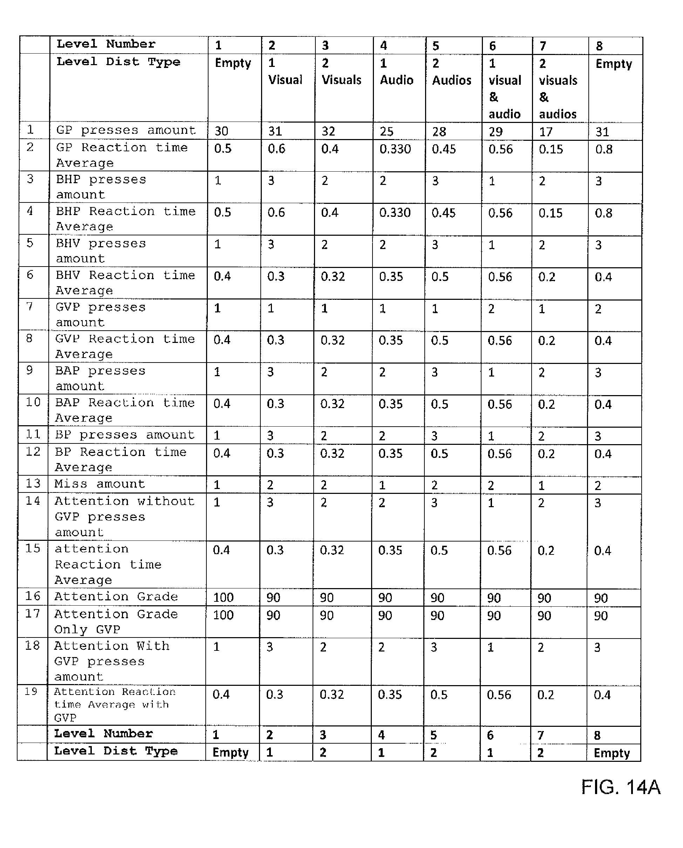

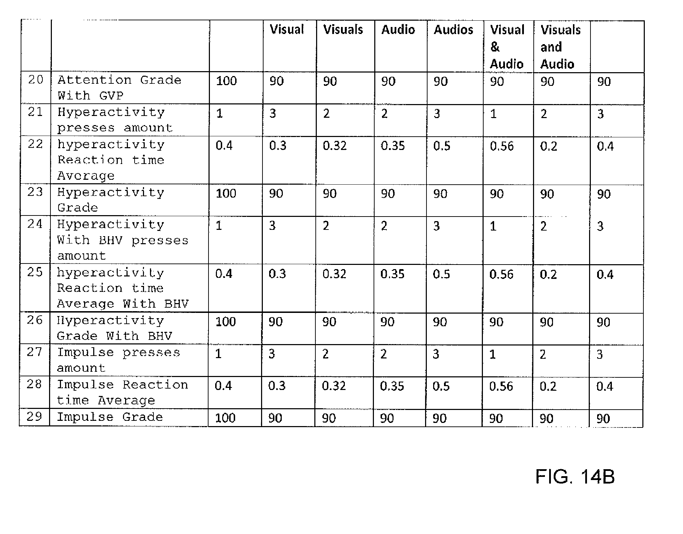

Figs. 14A - 14B, taken together, are an example of a table, also termed herein the mivSummaryByLvl table, useful for presenting information about Attention, hyperactivity and impulsiveness. Generally, Attention information may be derived from Good Presses, Good Void Presses and Miss states as defined in the state diagram of Fig. 12. Hyperactivity information may be derived from Bad Hyper Presses, Bad Hyper Void presses and Bad Presses states as defined in the state diagram of Fig. 12. Impulsiveness information may be derived from Bad Animal Presses states as defined in the state diagram of Fig. 12. As described above, for each press status the amount of presses is determined as well as the reaction time associated therewith.

Rows 1 - 13 of the table of Fig. 14A are examples of measures summarizing the response data collected from the end user whereas rows 14 onward are examples of operationalizations of psychological variables of interest.

Rows 1, 3, 5, 7, 9, 11 and 13 are totals of the number of occurrences of, respectively, the GP, BHP, BHV, GVP, BAP, BP and Miss states defined above and with reference to Fig. 12, for a particular end user's test session. Rows 2, 4, 6, 8, 10, 12 are, respectively, reaction time averages (or, for Row 2, raw reaction times, in which case Row 15 may store averages of Row 2) characterizing the GP, BHP, BHV, GVP, BAP and BP states respectively.

Regarding operationalizations, for example, Row 18 in the illustrated example is operationalized by summing rows 1 and 7, Row 19 in the illustrated example is operationalized by averaging rows 2 and 8, Row 21 in the illustrated example is operationalized by humming rows 3 and 11, Row 22 in the illustrated example is operationalized by averaging rows 3 and 11, Row 24 in the illustrated example is operationalized by summing rows 3, 5 and 11, Row 25 in the illustrated example is operationalized by averaging rows 3, 5 and 11, Row 30 in the illustrated example is operationalized by summing rows 5 and 7, and Row 31 in the illustrated example is operationalized by averaging rows 6 and 8. Row 23 in the illustrated example is operationalized by computing 100 - 10 x (line 3 + line 11). Row 26 in the illustrated example is operationalized by computing 100 - 10 x (line 3 + line 5 + line 11). Row 29 in the illustrated example is operationalized by computing 100 - 10 x (line 9).

Row 16 in the illustrated example is operationalized by applying a suitable grading functionality, such as that described below, to the values in row 1. Row 17 in the illustrated example is operationalized by applying a suitable grading functionality, such as that described below, to the values in row 7. Row 20 in the illustrated example is operationalized by applying a suitable grading functionality, such as that described below, to the sum of respective values in rows 1 and 7 respectively.

Some of the operationalizations in rows 14 onward may be identical to some of the measures in rows 1 - 13 (e.g., in the illustrated embodiments, rows 1 and 14 are identical, rows 9 and 27 are identical, rows 10 and 28 are identical). This type of implementation allows the psychological variable's operationalization to be configured; in a future implementation, the operationalizations of row 14 or 27 or 28 might be different.

It is appreciated that all measures and operationalizations appearing in the table of Figs. 14A - 14B are merely exemplary and are not intended to be limiting.

Figs. 13A - 13C are typically precursors of the table of Figs. 14A - 14B which are processed and soiled there-into.

Attention, hyperactivity and impulsiveness grades are computed for each level by a grading functionality e.g. using the following rules:
Hyperactivity and Impulsiveness grades may each start from an initial value such as 100 and include a penalty of, say, 10 points for each end user response, e.g. press, within "bad" categories. For example, Hyperactivity grades may include a penalty of, say, 10 points for each press within the following categories: Bad Hyper Presses, Bad Hyper Void presses and Bad Presses. Impulsiveness grades may include a penalty of, say, 10 points for each press within the Bad Animal Presses category.

In the illustrated embodiment, there are 33 targets per level; in this case, the grading functionality may be as follows:
33 GPs (good presses)→ Grade: 100
32 GPs → Grade: 95
31 GPs → Grade: 90
30 GPs → Grade: 80
29 GPs → Grade: 70
28 GPs → Grade: 60
27 GPs → Grade: 55
26 GPs → Grade: 50
25 GPs → Grade: 48
24 GPs → Grade: 46
23 GPs → Grade: 44
22 GPs → Grade: 42
26 GPs → Grade: 2
26 GPs → Grade: 0

It is appreciated that in the above example scheme, more points, e.g. 5 points are earned per good press if the number of good presses is above a certain threshold of, say, 25 good presses, whereas less points, e.g. only 2 points are earned if the number of good presses is below that threshold.

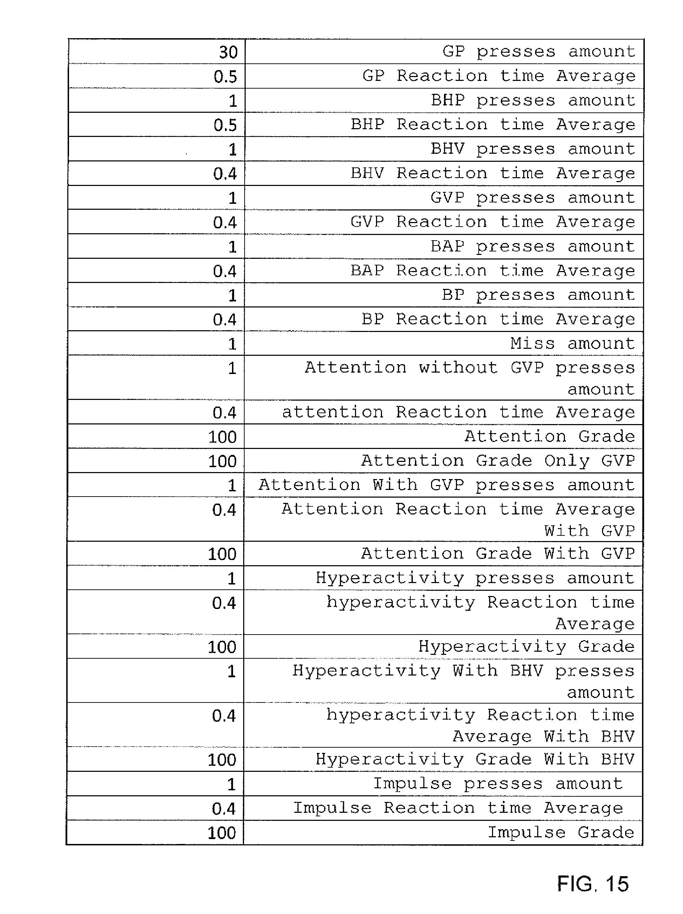

Fig. 15 is a table, termed herein the mivTopSummary table, which is similar to the table of Figs. 14A - 14B except that the grades are made from the top point of view of the test. As shown, instead of 8 (say) columns there is only one, whereas the fields typically stay the same.

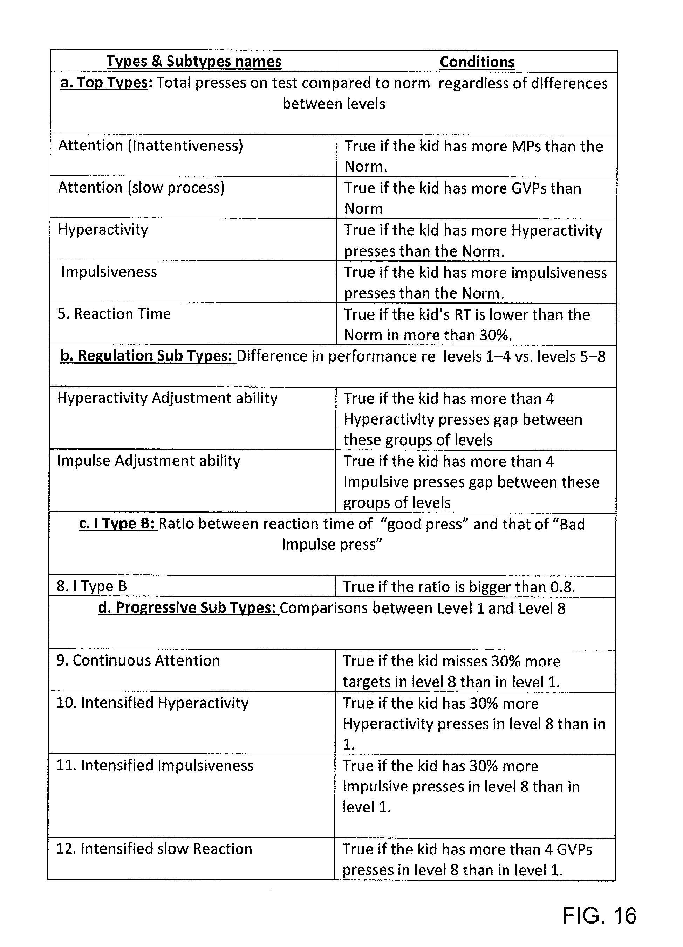

Typically, a pre-defined norm interval is available for each child and the child's test performance is compared to that norm e.g. as defined in the Types & Subtypes table of Fig. 16. The term "Hyperactivity presses" refers to the following sum: Bad Hyper Presses + Bad Hyper Void presses + Bad Presses. The term "Impulsiveness presses" refers to the total number of Bad Animal Presses. For each test, the user is indicated to have passed or not passed, either relative to a norm, or by comparing to a different level.

The tables of Figs. 13C - 16 may be stored as is on an online Database in association with ID particulars of the child end user.

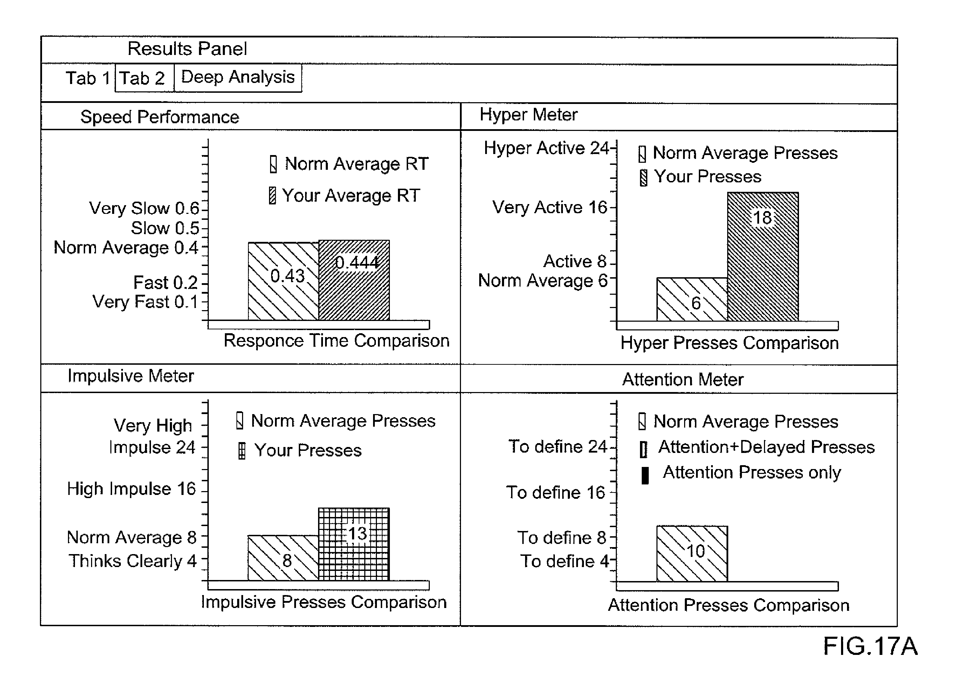

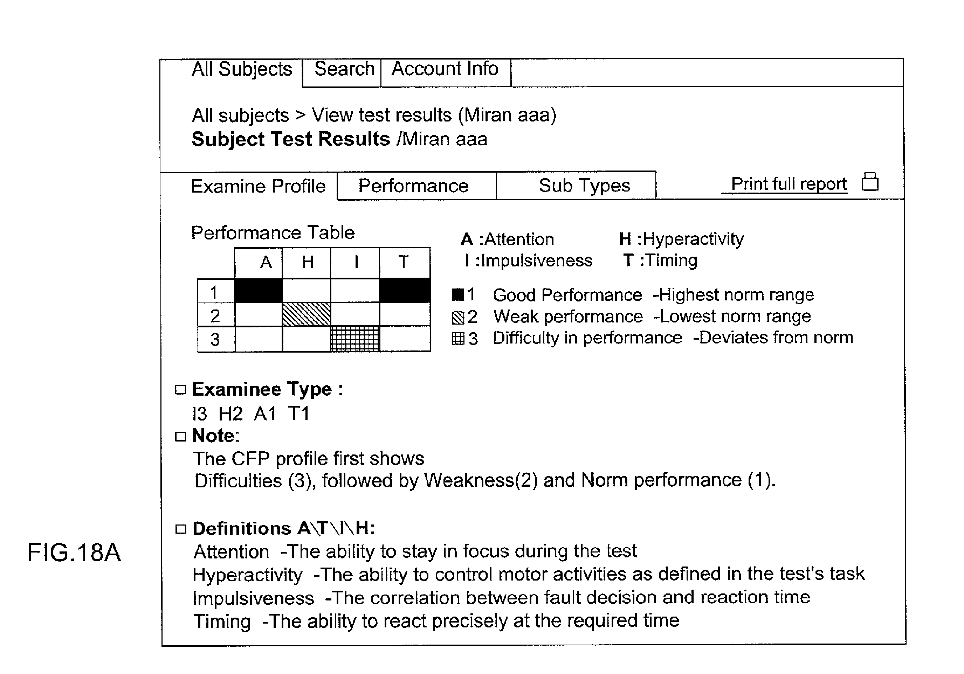

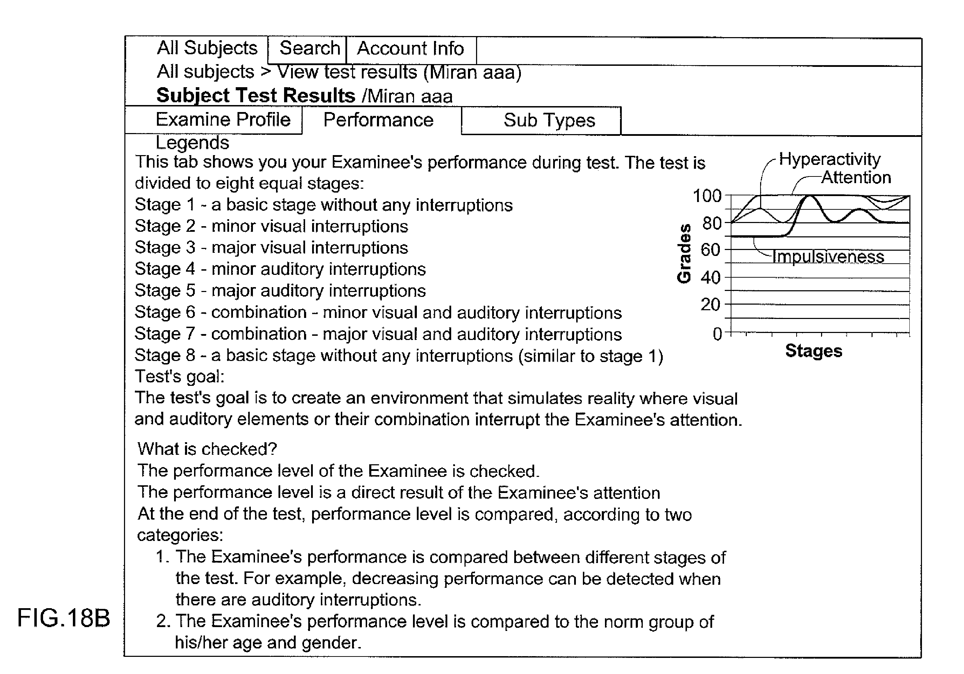

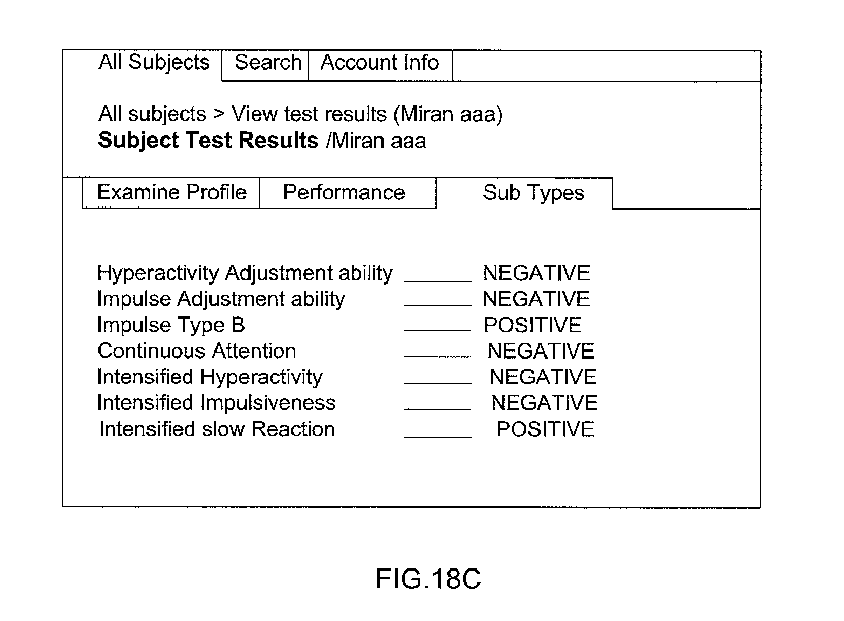

After the test is over and the data sent to the server a suitable summary is typically displayed. The display may for example include several tabs (tab1, ...tab4) e.g. as shown in Figs. 17A - 17D respectively. Another example output format includes the generally self-explanatory screen displays of Figs. 18A - 18C.

Tab 1, as shown in Fig. 17A, displays total test performance vs. the norm regardless of the differences between the levels. All of the data in this tab may relate to the mivTopSummary table of Fig. 15. The data may be presented in graph form e.g.:
Top left graph - speed Performance - shows the average reaction time.
Top right graph - Hyper meter - shows how many hyperactive presses occurred all along the test.
Bottom left graph - Impulse meter - shows how many Impulse presses occurred all along the test.
Bottom right graph - Attention meter - shows how many good presses occurred all along the test.

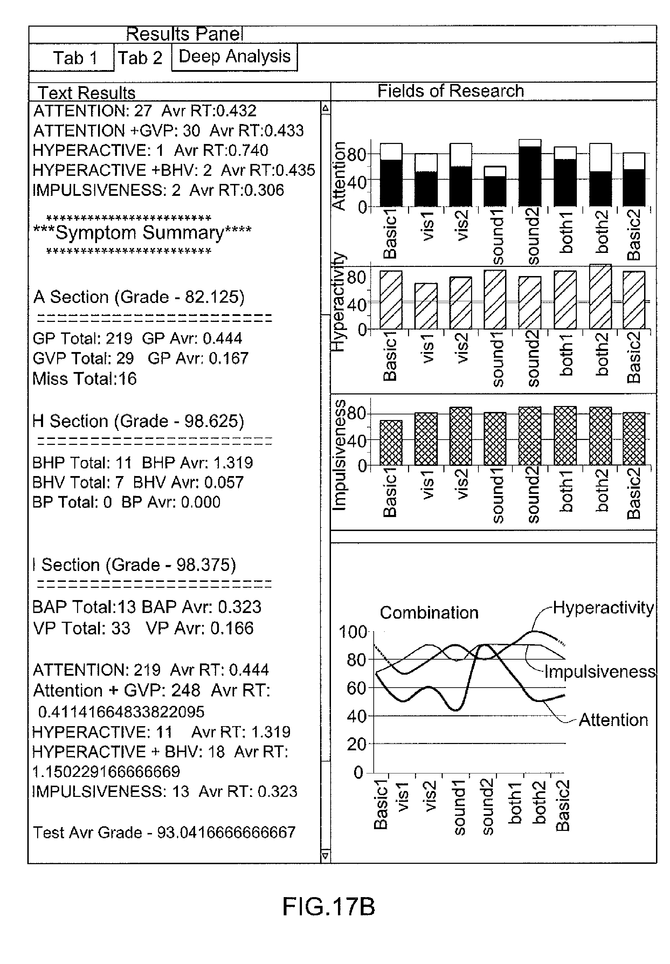

Tab 2, as shown in Fig. 17B, depicts information quantifying Performance in various fields, presented by levels, regardless of the norm. The information may include the following 3 sections:
a. Top right graph - level grades in the fields Attention Hyperactivity and Impulsiveness
b. Bottom right graph - top view on the same data as 1- allows us to see if one field from among Attention, Hyperactivity and Impulsiveness occurs at the expense of any other of the same fields.
c. Left Text Area - for each level all fields in Figs, 14A - 14B are printed out, in addition to the numbers and reaction times of certain fields (e.g. Attention, Hyperactivity and Impulsiveness) from the table of Fig. 15.

The data in the first and second sections typically relates to the mivSummaryByLvl table of Figs. 14A - 14B. The data in the third section typically relates to the mivSummaryByLvl and mivTopSummary tables of Figs. 14A -15.

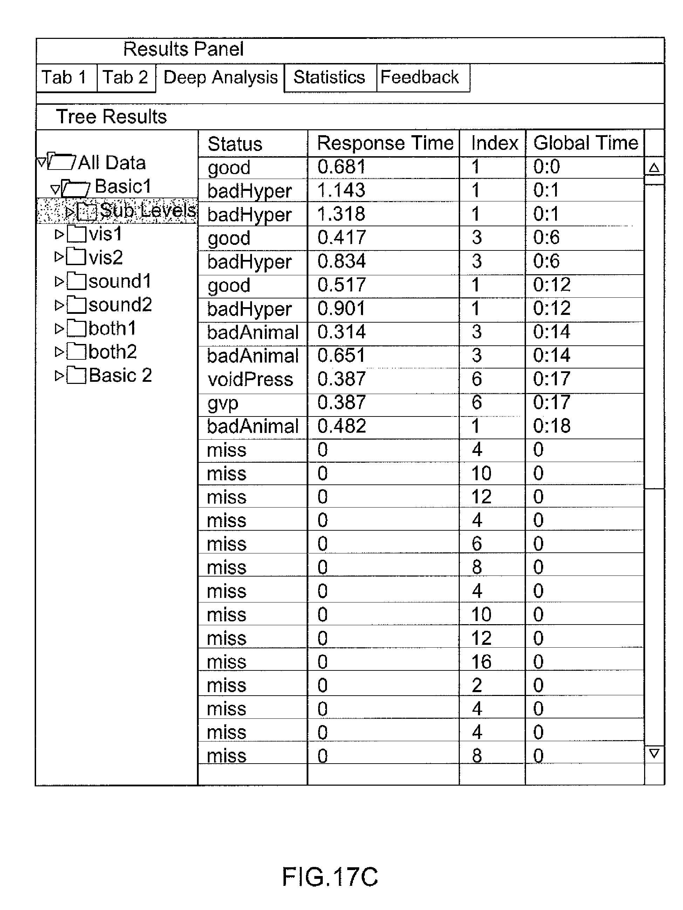

Tab 3, as shown in Fig. 17C, provides an "All presses report" including information related to the mivRawResults table of Fig. 13C. This tab allows an analyst user to see and navigate between all of the presses made throughout test time. For each press/miss the Status, Response time, Index in sub level, and Global Test Time can typically be seen.

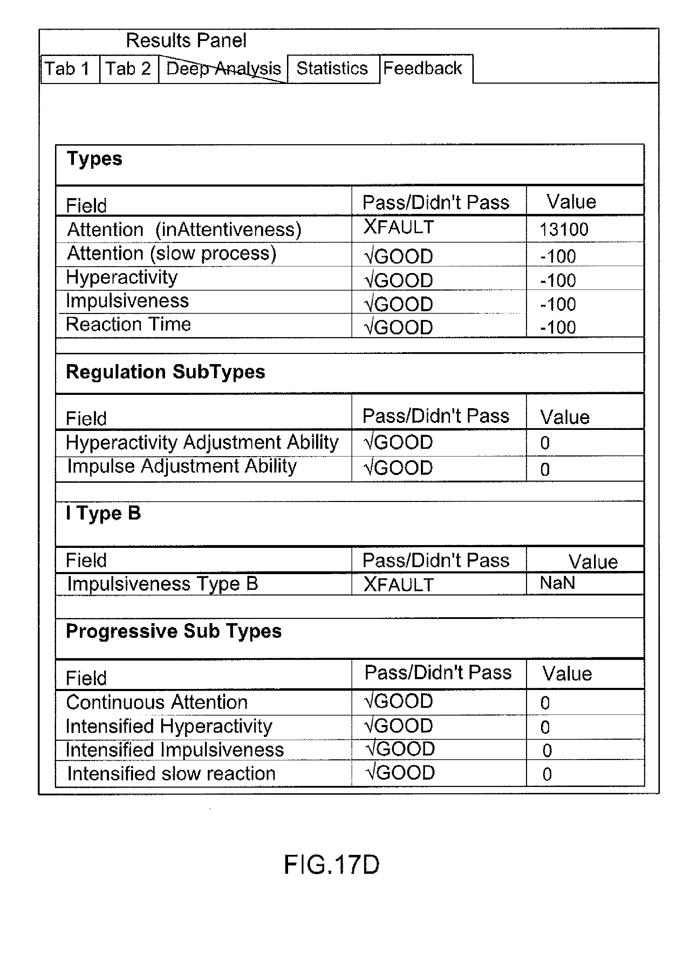

Tab 4, as shown in Fig. 17D, declares types and subtypes that have been found. The information displayed relates to the Types & Subtypes table of Fig. 16. For each of a plurality of patterns deemed of interest, there is a correlate row. For each pattern the system determines whether the child end user passed or did not pass and the actual value of the comparison is typically presented. Each user's performance is typically analyzed and searched for 12 specific kinds of behaviors that correlate to each row in the types tab as shown in Fig. 17D.

It is appreciated that the system shown and described above is merely exemplary. An example of a suitable method of operation of the system shown and illustrated herein may include the following steps:
a. Raw performance data is gathered along all of the 8 levels, each of which includes 11 sublevels, by recording each change of state, each miss and each type of press being defined as per the state chart of Fig. 12. For each change of state, record time on a global timer initialized typically at the beginning of each test session.
b. Sorting & analyzing stage typically occurring at the end of test (after the 8th level in the illustrated embodiment). Generate data e.g. by generating the main data table of Fig. 14A.
c. Compute diagnoses e.g. using Fig16
d. Present outputs characterizing the end user's cognitive functions, e.g. using any of the display functionalities of Figs. 17A - 18D.

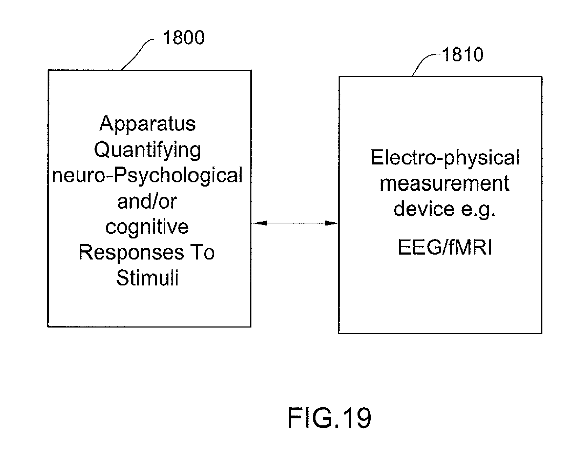

According to certain embodiments of the present invention, as shown in Fig. 19, a testing system 1800 shown and described herein is integrated with an electro-physical measurement device 1810 such as an E.E.G or fMRI device. The result is a system for deeper evaluation because a user's impulsive action or hyperactive action can be correlated temporally to occasions on which the user's EEG or FMRI showed signs of brain activity. It is appreciated that the data flow between the testing system 1800 and electro-physical measurement device 1810 is typically bi-directional in that data generated by the testing system 1800 can be used as input to enhance operation of or output of the EEG, functional MRI or other electro-physical measurement device and conversely, data generated by the EEG, functional MRI or other electro-physical measurement device can be used as input to enhance operation of or output of the testing system 1800.

It is appreciated that terminology such as "mandatory", "required", "need" and "must" refer to implementation choices made within the context of a particular implementation or application described herewithin for clarity and are not intended to be limiting since in an alternative implantation, the same elements might be defined as not mandatory and not required or might even be eliminated altogether.

It is appreciated that software components of the present invention including programs and data may, if desired, be implemented in ROM (read only memory) form including CD-ROMs, EPROMs and EEPROMs, or may be stored in any other suitable computer-readable medium such as but not limited to disks of various kinds, cards of various kinds and RAMs. Components described herein as software may, alternatively, be implemented wholly or partly in hardware, if desired, using conventional techniques. Conversely, components described herein as hardware may, alternatively, be implemented wholly or partly in software, if desired, using conventional techniques.

Included in the scope of the present invention, inter alia, are electromagnetic signals carrying computer-readable instructions for performing any or all of the steps of any of the methods shown and described herein, in any suitable order; machine-readable instructions for performing any or all of the steps of any of the methods shown and described herein, in any suitable order; program storage devices readable by machine, tangibly embodying a program of instructions executable by the machine to perform any or all of the steps of any of the methods shown and described herein, in any suitable order; a computer program product comprising a computer useable medium having computer readable program code, such as executable code, having embodied therein, and/or including computer readable program code for performing, any or all of the steps of any of the methods shown and described herein, in any suitable order; any technical effects brought about by any or all of the steps of any of the methods shown and described herein, when performed in any suitable order; any suitable apparatus or device or combination of such, programmed to perform, alone or in combination, any or all of the steps of any of the methods shown and described herein, in any suitable order; electronic devices each including a processor and a cooperating input device and/or output device and operative to perform in software any steps shown and described herein; information storage devices or physical records, such as disks or hard drives, causing a computer or other device to be configured so as to carry out any or all of the steps of any of the methods shown and described herein, in any suitable order; a program pre-stored e.g. in memory or on an information network such as the Internet, before or after being downloaded, which embodies any or all of the steps of any of the methods shown and described herein, in any suitable order, and the method of uploading or downloading such, and a system including server/s and/or client/s for using such; and hardware which performs any or all of the steps of any of the methods shown and described herein, in any suitable order, either alone or in conjunction with software.

Any computations or other forms of analysis described herein may be performed by a suitable computerized method. Any step described herein may be computer-implemented. The invention shown and described herein may include (a) using a computerized method to identify a solution to any of the problems or for any of the objectives described herein, the solution optionally include at least one of a decision, an action, a product, a service or any other information described herein that impacts, in a positive manner, a problem or objectives described herein; and (b) outputting the solution.

Features of the present invention which are described in the context of separate embodiments may also be provided in combination in a single embodiment. Conversely, features of the invention, including method steps, which are described for brevity in the context of a single embodiment or in a certain order may be provided separately or in any suitable subcombination or in a different order. "e.g." is used herein in the sense of a specific example which is not intended to be limiting. Devices, apparatus or systems shown coupled in any of the drawings may in fact be integrated into a single platform in certain embodiments or may be coupled via any appropriate wired or wireless coupling such as but not limited to optical fiber, Ethernet, Wireless LAN, HomePNA, power line communication, cell phone, PDA, Blackberry GPRS, Satellite including GPS, or other mobile delivery. It is appreciated that in the description and drawings shown and described herein, functionalities described or illustrated as systems and sub-units thereof can also be provided as methods and steps therewithin, and functionalities described or illustrated as methods and steps therewithin can also be provided as systems and sub-units thereof. The scale used to illustrate various elements in the drawings is merely exemplary and/or appropriate for clarity of presentation and is not intended to be limiting.

## Claims

1. A method for measuring effects of distraction on a human subject, the method comprising:
presenting at least one stimulus, at least one visual distraction and at least one audio distraction to a subject;
measuring at least one aspect of at least one of the subject's commissions and omissions responsive to said stimulus;
computing an indication of quality of at least one of the subject's commissions and omissions when said visual distraction is present; and
computing an indication of quality of at least one of the subject's commissions and omissions when said audio distraction is present.

2. A method according to claim 1 and also comprising:
computing an indication of quality of at least one of the subject's commissions and omissions when said audio and visual distractions are both present.

3. A method according to any of claims 1 - 2 and also comprising using at least one of said indications to quantify attentiveness.

4. A method according to any of claims 1 - 3 and also comprising using at least one of said indications to quantify hyperactivity.

5. A method according to claim 1 wherein said measuring comprises generating a reaction time parameter representing the subject's reaction time and measuring at least one aspect of at least one of the subject's commissions and omissions responsive to said stimulus, other than reaction time,
and wherein said computing comprises generating an output characterizing the subject as a function of said reaction time parameter and of said aspect other than reaction time.

6. A system for measuring effects of distraction on a human subject, the system comprising:
a presentation generator operative to present to a subject at least one stimulus accompanied by at least one distraction selected from a universe of distractions including at least one visual distraction and at least one audio distraction; and
a performance measurement unit operative to measure the subject's test performance as affected by at least one audio distraction, to measure the subject's test performance as affected by at least one visual distraction, and to compare the two performances thereby to characterize the subject's differential response to audio and visual distractions.

7. A system according to claim 6 wherein said performance measurement unit is also operative to measure the subject's test performance as affected by at least one audio distraction in combination with at least one video distraction.

8. A system according to any of claims 6 - 7 wherein said performance measurement unit is also operative to measure at least one aspect of at least one of the subject's commissions and omissions responsive to said stimulus.

9. A system according to any of claims 6 - 8 wherein said performance measurement unit is also operative to compute an indication of quality of at least one of the subject's commissions and omissions when said visual distraction is present.

10. A system according to any of claims 6 - 9 wherein said performance measurement unit is also operative to compute an indication of quality of at least one of the subject's commissions and omissions when said audio distraction is present.

11. A system according to any of claims 6 - 10 wherein said universe of distractions includes a plurality of visual distractions with a corresponding plurality of profiles of visual characteristics.

12. A system according to any of claims 6 - 11 wherein said universe of distractions includes a plurality of audio distractions with a corresponding plurality of profiles of audio characteristics.

13. A system according to claim 11 wherein said performance measurement unit is operative to compare the subject's performance as affected by a visual distraction with a first profile of visual characteristics, with the subject's performance as affected by a visual distraction with a second, different profile of visual characteristics.

14. A system according to claim 12 wherein said performance measurement unit is operative to compare the subject's performance as affected by a audio distraction with a first profile of audio characteristics, with the subject's performance as affected by a audio distraction with a second, different profile of audio characteristics.

15. A method according to claim 5 and wherein said aspect other than reaction time comprises at least one of:
an aspect of the order of the subject's commissions and omissions;
an aspect of the relative timing of the subject's commissions and omissions; and
timing of commissions and omissions relative to a void time period in which said stimulus is not presented to the subject.
